# FASCICULE DE BREVET EUROPEEN

(11) **EP 1 517 888 B1**
(45) Date de publication et mention de la délivrance du brevet: **14.05.2008**
(21) Numéro de dépôt: 03761633.1
(22) Date de dépôt: 24.06.2003
(51) Int. Cl.: C07C 319/14, C07C 323/03, C07C 303/16, C07C 309/80

(54) **PROCEDE DE SYNTHESE DE DERIVES A RADICAL HYDROGENOFLUOROMETHYLENESULFONYLE**
VERFAHREN ZUR HERSTELLUNG VON HYDROGENOFLUORMETHYLENSULFONYL-DERIVATEN
METHOD FOR SYNTHESIS OF HYDROGENOFLUOROMETHYLENESULPHONYL RADICAL DERIVATIVES

(30) Priorité: 28.06.2002 FR 0208090
(43) Date de publication de la demande: 30.03.2005
(73) Titulaire: RHODIA CHIMIE, 92512 Boulogne Billancourt Cedex (FR)
(72) Inventeur: SAINT-JALMES, Laurent, F-69330 Meyzieu (FR)
(74) Mandataire: Dutruc-Rosset, Marie-Claude
(86) Numéro de dépôt international: PCT/FR2003/001940
(87) Numéro de publication internationale: WO 2004/002951

(56) Documents cités:
- G.G.I. MOORE, ET AL.: "Fluoroalkanesulphonyl chlorides" JOURNAL OF ORGANIC CHEMISTRY, vol. 44, no. 10, 11 mai 1979 (1979-05-11), pages 1708-1711, XP002952300 American Chemical Society, Washington, DC, US ISSN: 0022-3263
- B.R. LANGLOIS: "Improvement of the synthesis of aryl difluoromethyl ethers and thioethers by using a solid-liquid phase-transfer technique" JOURNAL OF FLUORINE CHEMISTRY, vol. 41, no. 2, novembre 1988 (1988-11), pages 247-261, XP002232989 Elsevier Sequoia, Lausanne, CH ISSN: 0022-1139
- R. FELDHOFF, ET AL.: "Darstellung und Eigenschaften Trifluomethyl- und Trifluomethylchalkogenyl-substituierter Adamantane" JOURNAL OF FLUORINE CHEMISTRY, vol. 67, no. 3, juin 1994 (1994-06), pages 245-251, XP002232990 Elsevier Sequoia, Lausanne, CH ISSN: 0022-1139

## Description

La présente invention a pour objet une nouvelle synthèse de dérivés à radical hydrogénofluorométhylènesulfonyle ou sulfinyle.

Elle vise plus particulièrement la synthèse de dérivés de l'acide difluorométhanesulfinique ou sulfonique, et notamment l'acide proprement dit, ses sels et ses chlorures d'acide.

Les acides sulfoniques portés par des groupes électroattracteurs et notamment portés par des groupes électro-attracteurs dont le carbone porteur de la fonction sulfonique porte également au moins un fluor sont des composés de plus en plus intéressants, d'une part, parce qu'ils permettent de donner des propriétés particulières spécifiques aux médicaments ou aux dérivés pour l'agriculture et, d'autre part, parce qu'ils peuvent servir d'éléments constitutifs de sels pour les batteries, notamment les batteries dites au lithium.

Parmi les produits intéressants, il convient de signaler notamment les halogénures de sulfonyle et de sulfinyle. Parmi les halogénures, on peut citer le bromure, relativement instable, le chlorure et le fluorure.

Les procédés d'obtention de ces composés sont en général longs et difficiles ou donnent des rendements relativement faibles, ou bien nécessitent l'utilisation de solvants ou de conditions opératoires particulièrement onéreuses.

Si l'on se réfère plus spécifiquement à l'acide difluorométhanesulfonique, parfois appelé acide diflique, sa synthèse est peu décrite dans la littérature et nécessite l'emploi de solvants relativement coûteux et nécessite la formation de sels en grande quantité.

Il est connu selon G. G. I. Moore [Journal of Organic Chemistry, 1979, 44 (10), 1708-1711] de préparer du chlorure de difluorométhanesulfonyle par réaction du benzylthiolate de sodium avec du chlorodifluorométhane dans un mélange de dioxane et d'eau suivi par un oxydation au chlore. Dans le procédé décrit, le rapport massique entre le dioxane et l'eau est de ca 5 :1.

C'est pourquoi un des buts de la présente invention est de fournir un procédé de synthèse des acides, ou des dérivés d'acide à radical hydrogénofluorométhylènesulfonyle, ou sulfinyle, qui permettent d'éviter l'utilisation de solvants coûteux instables ou difficiles à mettre en oeuvre.

Un autre but de la présente invention est de fournir une réaction du type précédent qui permette d'obtenir de bons rendements et notamment des rendements de transformation (RT, c'est-à-dire le rendement en produit désiré obtenu par rapport à la quantité de produit de départ consommé) élevés, c'est-à-dire supérieurs à 60%, de préférence supérieurs à 70%.

Un autre but de la présente invention est de fournir un procédé de nature polyvalente de manière qu'il puisse conduire aussi bien à la formation de sels d'acides, d'acides, ou d'halogénures d'acides.

Il est également souhaitable que l'on puisse obtenir essentiellement l'acide correspondant au radical sulfonique.

Un autre but de la présente invention est de fournir un procédé qui permette l'obtention de dérivés de nature sulfinique.

Ces buts, et d'autres qui apparaîtront par la suite, sont atteints au moyen d'un procédé de synthèse de dérivés à radical hydrogénofluorométhanesulfonyle ou sulfinyle comportant au moins les étapes suivantes :
a) une étape de condensation dans un solvant d'un thiolate (c'est-à-dire un sel de sulfure de monoalcoyle), dont le contrecation (c'est-à-dire le cation assurant la neutralité électrique de la molécule) est avantageusement non basique (c'est-à-dire un cation dont l'hydrolyse n'intervient pour des concentrations de 10⁻²-N qu'à des pH supérieurs à 10, avantageusement à 12), avec un composé présentant un carbone d'hybridation sp³ porteur d'un hydrogène, d'un fluor, d'un halogène lourd choisi parmi le chlore, le brome et l'iode, et d'un groupe électro-attracteur choisi parmi le fluor et ceux dont le dont le σₚ est au moins égal à 0,2 ; avantageusement à 0,4 ;
b) une étape d'oxydation, avantageusement d'halogénation, de préférence de chloration ou de bromation, en présence d'une phase aqueuse ;
ledit solvant de l'étape a) étant choisi parmi les solvants non miscibles à l'eau, parmi les phases aqueuses et parmi la combinaison biphasique d'un solvant non miscible à l'eau et d'une phase aqueuse, lesdites phases aqueuses comportant au plus 1/3 en masse de solvant non aqueux miscible à l'eau; le rapport entre la quantité en équivalent du thiolate et la quantité d'eau en mole étant au plus égal à 50.

Lorsque l'on utilise une phase aqueuse comportant un solvant non aqueux miscible à l'eau, il est préférable que ce solvant ne soit pas basosensible, c'est-à-dire qu'il ne soit pas détruit par la présence de base, en particulier les amides et les esters solubles sont, dans la mesure du possible, à éviter. Par l'expression "miscible à l'eau", il convient de comprendre un solvant miscible en toute proportion avec l'eau. Les solvants non miscibles à l'eau sont avantageusement choisis parmi ceux qui présentent une solubilité dans l'eau, en masse, d'au plus 10%, c'est-à-dire que l'eau puisse dissoudre au plus 10%, dans les conditions normales de température et de pression, du solvant non miscible à l'eau. Comme solvant non miscible à l'eau, on peut utiliser les dérivés aromatiques chlorés, voire certains éthers.

Il est également préférable que la solubilité dans l'eau dudit solvant non miscible soit au plus 5% en masse, de préférence 2% en masse.

Parmi les solvants non miscibles, on peut faire une mention particulière des dérivés aromatiques, éventuellement substitués et notamment les dérivés halogénoaromatiques, tels que les mono di- ou tri-chlorobenzènes.

L'anisole est susceptible, comme les autres éthers de phénol, d'être intéressant. Toutefois, l'activation par l'oxygène, ou par noyau aromatique hydrogène de nature aliphatique peut gêner dans la deuxième étape et dans ce cas là, il conviendra d'éliminer le solvant avant l'étape dite d'halogénation.

Toutefois, selon la présente invention, il est préférable d'utiliser comme solvant une phase aqueuse comportant peu ou, de préférence, pas de solvant organique miscible, mais pouvant comporter des sels dissous et notamment des bases hydrosolubles.

Un point important de la présente invention est que pour maximiser le rendement, il est très préférable d'utiliser une faible quantité d'eau. Ainsi, il est préférable que le rapport entre la quantité, exprimée en équivalent, du thiolate et la quantité d'eau exprimée en mole, soit au plus égal à 30, avantageusement à 20.

On exprime la quantité de thiolate en équivalent pour tenir compte du cas où l'on utiliserait des composés comportant deux fonctions sulfure, ou deux fonctions thiolate. Il est même envisageable, quoique sans doute peu intéressant économiquement, d'utiliser des composés à multiples fonctions thiol. Selon la présente invention, il est préférable que la quantité d'eau soit suffisamment importante pour former une phase significative. Ainsi, le rapport entre la quantité en équivalent du sulfure d'alcoyle, et la quantité d'eau (dans la ou les phases basiques) exprimée en mole, soit au moins égal à 0,5, avantageusement à 1, de préférence à 1,5.

Selon la présente invention, il est préférable que lesdites phases aqueuses comportent au plus un quart en masse de solvant non aqueux, de préférence au plus un dixième.

Il est également préférable que le rapport molaire [H₂O]/([H₂O]+[solvant miscible à l'eau]) soit au moins égal à 0,9, avantageusement à 0,95. Enfin, il est préférable de limiter la concentration de la base dans la phase aqueuse à 0,5 équivalent par kg de phase aqueuse.

Il convient de signaler que le thiolate cationique qui constitue un des substrats de l'étape A peut être fait in situ par action d'un sulfure R-SH sur une base plus forte que R-S⁻.

La limitation sur la concentration de la base, et notamment de OH⁻, ne vise que l'excès de base et donc ne tient pas compte de la quantité de base consommée pour faire le thiolate.

Le thiolate, peut être écrit sous la formule générale R-S-M où M correspond à un métal, ou à un cation qui, associé avec l'anion OH⁻, constitue une base forte, c'est-à-dire une base dont l'acide associé présente un pKa au moins égal à 10, avantageuse à 12, de préférence à 14.

La présente invention peut, dans certains cas, surtout lorsqu'il y a une phase organique comportant un solvant organique non miscible à l'eau, un agent de transfert de phase, ces agents sont bien connus de l'homme du métier et peu en particulier être choisis parmi les oniums et parmi les iniums, parmi les éther-couronnes ou les éthers cryptants tels que la TDA1 (N[CH₂-CH₂-O-CH₂-CH₂]₃N). Selon la présente invention, il est préférable de travailler avec une faible quantité (ou excès par rapport à la quantité nécessaire pour neutraliser le thiol en thiolate) de base dans la phase aqueuse, cette quantité étant avantageusement au moins égale à 5%, avantageusement à 10%, de la quantité dudit thiolate (R-S-M).

Il est toutefois préférable que le milieu aqueux de l'étape a) comporte une quantité de base forte pKa au plus égale à une fois la quantité dudit thiolate, de préférence au plus 50%, plus préférentiellement au plus 30%.

Selon la présente invention, il a été montré qu'il était souhaitable d'utiliser des sulfures portés par un alcoyle qui soit tertiaire ou de nature benzylique ou allylique.

Cette activation du carbone porteur du soufre permet de faciliter la coupure carbone-soufre qui est réalisée dans l'étape b). Toutefois, le radical allylique étant susceptible de donner des polymérisations et des réactions parasites gênantes, il est préférable de se cantonner aux dérivés alcoyles de nature tertiaire ou benzylique.

Il est également possible d'exprimer la limitation en solvant organique polaire miscible à l'eau en indiquant que le rapport molaire entre la quantité d'un éventuel solvant polaire miscible, exprimée en mole, et la somme, exprimée en équivalent, des cocations du sulfure et de la base éventuelle, soit avantageusement au plus égal à 1, de préférence au plus égal à 1,5, plus préférentiellement au plus égal à 1 /10.

Parmi les composés intéressants à synthétiser, on peut citer les composés radicaux hydrogénofluorométhylène de formule I et IV'

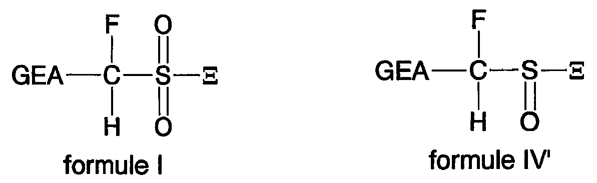

Dans cette formule, GEA est un groupe électro-attracteur choisi parmi le fluor et ceux dont le σₚ (constante de Hammett) est au moins égal à 0,2, avantageusement à 0,4, ou Ξ représente soit un halogène, avantageusement choisi parmi le chlore et le brome, soit un oxygène porteur lui-même soit d'un hydrogène, soit d'une charge négative.

La réaction de l'étape peut être écrite comme suit

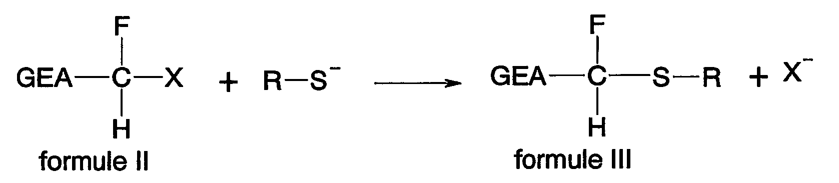

Dans les formules ci-dessus, R représente un alcoyle au sens étymologique du terme, c'est-à-dire un alcool dont on a éliminé la fonction OH.

La réaction d'halogénation qui constitue un mode préféré de mise en oeuvre de l'étape b) peut être symbolisée par l'une ou plusieurs des réactions ci-après :

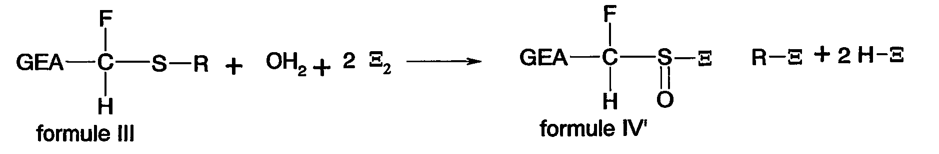

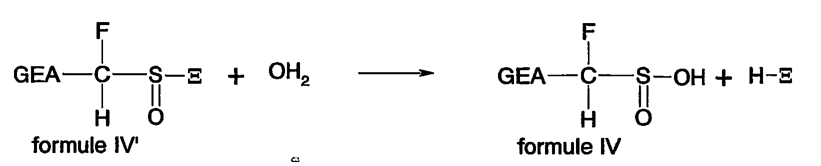

Dans cette réaction, l'on constate que l'halogène, symbolisé ici par Ξ₂, oxyde le soufre, libère le radical alcoyle sous forme d'un halogénure d'alcoyle R-Ξ et libère trois acides H-Ξ. Cette réaction conduit à un dérivé sulfinique. Ce dérivé sulfinique figuré dans cette équation est l'acide sulfinique lui-même. D'une part, il est possible de récupérer le dérivé intermédiaire de IV' qui peut constituer un réactif de valeur et, d'autre part, la réaction ne s'arrête pas à cette étape, sauf si on limite la quantité d'halogène car l'acide sulfinique est oxydé en dérivés sulfoniques ; ce dérivé peut être soit un halogénure de sulfonyle, selon la réaction suivante : 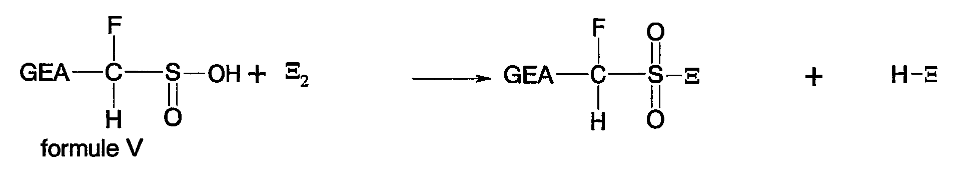 ou bien, suivant les conditions opératoires, elle peut conduire à l'acide sulfonique ou ses sels, et ce, selon la réaction ci-après :

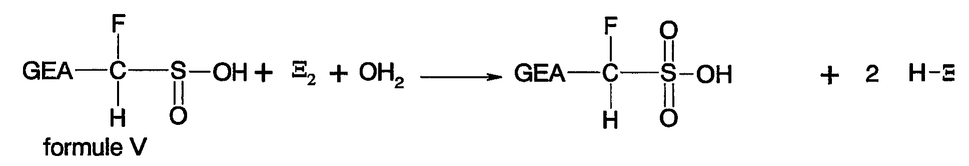

Selon la présente invention, il a pu être montré que l'on pouvait choisir les conditions opératoires pour optimiser la réaction soit vers un halogénure de sulfonyle, soit vers l'acide sulfonique ou ses sels.

Ainsi, lorsque l'on désire obtenir un halogénure d'acide, il est souhaitable que l'étape b) soit menée en présence d'un sel dissocié dissous dans la phase aqueuse, avantageusement en quantité suffisante, pour atteindre et/ou dépasser une concentration de 1 N, de préférence 2N (en anion), avantageusement l'anion est un halogénure, de préférence l'halogénure correspondant à l'halogénure d'acide que l'on désire. Ceci est particulièrement vrai lorsque l'halogénure d'acide désiré est le chlorure d'acide et le chlorure de sulfonyle. Lorsque l'on utilise un halogénure de rang atomique supérieur à celui de l'halogène utilisé, bien entendu, l'on forme comme halogénure de sulfonyle celui correspondant à l'halogénure de rang le plus élevé dans le milieu.

Une autre caractéristique favorisant la formation de l'halogénure d'acide est le maintien à un pH situé dans un intervalle allant de 4 à 9, avantageusement de 5 à 8. Ces deux conditions opératoires n'étant pas antagonistes, il est possible de les combiner et donc d'obtenir un bon résultat en utilisant une solution saline à un pH allant de 4 à 9.

Il est préférable que la solution saline spécifiée ci-dessus soit une solution d'un halogénure alcalin (bromure et de préférence chlorure, mais non iodure) à une concentration au moins 1N, de préférence au moins 2N en halogénure.

Si l'on désire privilégier la formation d'acide sulfonique, il est préférable de se placer alors à des pH acides, c'est-à-dire des pH au plus égaux à 2, de préférence au plus égaux à 1, de préférence aux alentours de 0, et d'éviter de réaliser la chloration ou l'hydrolyse en milieu salin. Il convient ainsi d'éviter une teneur en milieu salin supérieure à 1N. Il est également souhaitable, pour abaisser la teneur saline, de séparer la phase aqueuse de la phase sulfure qui se forme en général lors de l'étape a). Lorsque cette étape de sulfure ne s'est pas formée, il est alors souhaitable de faire une extraction liquide-liquide ou liquide-solide pour récupérer le sulfure, chasser le solvant, et ensuite traiter la phase sulfure dans une phase aqueuse de manière à réaliser le clivage entre l'atome de soufre et l'atome de carbone de l'alcoyle lié au soufre.

Un des intérêts de la présente invention est de pouvoir réaliser la synthèse d'un halogénure (bromure et avantageusement chlorure) en une seule étape sans changer de milieu, en réalisant la réaction dans un milieu aqueux concentré à l'étape a) et en reprenant le milieu aqueux après éventuellement élimination des solvants miscibles ou non miscibles et clivage avec formation d'un groupement sulfinyle ou sulfonyle comme décrit dans l'étape b).

La température de réaction est avantageusement comprise entre 50°C et 110°C, de préférence entre 60°C et 90°C.

La pression est relativement élevée en raison du fait que souvent les composés présentant un carbone d'hybridation sp³ porteur d'un hydrogène, d'un fluor, et d'un halogène lourd, sont volatils, comme c'est le cas quand GEA est fluor et que ledit composé et le composé utilisé comme réfrigérant sous le nom de R-22 (c'est-à-dire le chlorodifluorométhane) sont très volatils, ce qui conduit à une pression élevée pour maintenir un minimum de ces composés dans une phase liquide et surtout aqueuse.

Selon un mode avantageux de l'invention, le groupe électro-attracteur, tel que celui qui est figuré par GEA dans les équations précédentes, est un groupe hautement fluoré, avantageusement le nombre total de carbone de GEA (groupe électro-attracteur), plus spécifiquement Rf, est compris entre 1 et 15, de préférence entre 1 et 10.

Ainsi le groupe électroattracteur est avantageusement choisi parmi le fluor et les groupes Rf ; par Rf on entend un radical de formule :

GEA'-(CX₂)ₚ-

- où les X, semblables ou différents, représentent un chlore, un fluor ou un radical de formule CₙF₂ₙ₊₁, avec n entier au plus égal à 5, de préférence à 2, avec la condition qu'au moins un des X soit fluor, fluor avantageusement porté par le carbone relié au soufre ;
- où p représente un entier au plus égal à 2 ;
- où GEA' représente un groupe électroattracteur (c'est à dire σₚ supérieur à zéro, avantageusement à 0,1, de préférence à 0,2) dont les éventuelles fonctions sont inertes dans les conditions de la réaction, avantageusement fluor ou un reste perfluoré de formule CₙF₂ₙ₊₁ avec n entier au plus égal à 8, avantageusement à 5.

Ainsi qu'il a déjà été mentionné, une des mises en oeuvre préférées de l'invention est celle qui correspond au cas où le groupe électroattracteur est le fluor, ce qui implique que le composé est le R-22, c'est-à-dire le chlorodifluorométhane.

Dans la première étape, l'étape a), il est souhaitable qu'il y ait un minimum de quantité d'eau, et ce minimum peut être défini de la manière ci-après. Le rapport entre l'eau exprimée en mole et le cation, plus exactement la somme des cations présents, exprimée en équivalent, est au moins égal à 4, avantageusement à 6, de préférence à 8, pour éviter que le milieu ne soit trop polaire et gêne la solubilité du substrat comportant un carbone d'hybridation sp³ porteur d'un hydrogène, d'un fluor et d'un hydrogène lourd.

Avantageusement, le ou les cations présents dans la phase aqueuse, sont des cations monovalents. Il est préférable que ces cations soient choisis parmi les phosphoniums quaternaires, les ammoniums quaternaires, et les cations alcalins, et, avantageusement ces derniers, et plus préférentiellement parmi ces derniers, les cations correspondant au sodium et correspondant au potassium.

Pour obtenir une cinétique satisfaisante, il est préférable que la température soit au moins égale à 80°C dans l'étape a).

Au cours de la réaction, l'halogénure formé R-Ξ est récupéré puis soumis à un sulfure alcalin pour reformer le produit de départ, à savoir le sulfure mixte d'alcoyle et de cation.
Les exemples non limitatifs suivants illustrent l'invention.

### Exemple 1 - Synthèse du sulfure de benzyle et de difluorométhyle

Un équivalent de soude à 40% en masse dans l'eau a été mélangé à un équivalent de benzylmercaptan avec du R-22 (1,1 équivalent molaire) à 60°C pendant 1 h. Les essais sont réalisés dans un réacteur de 500 ml en Hastalloy à une pression autogène à 60°C. La pression est de 3,5 bars. Sur deux essais réalisés dans les mêmes conditions, on obtient un rendement chimique de 75% par dosage par RMN sur l'isotope 19 du fluor.

### Exemple 2 - Variation des différents paramètres de la synthèse du sulfure de benzyle et de difluorométhyle à partir du benzylmercaptan

Le mode opératoire décrit ci-dessus a été repris en modifiant la durée et en introduisant un large excès de R-22 (chlorodifluorométhane) progressivement. La réaction a été faite à pression atmosphérique pendant une durée de 4 h.

Les détails des conditions opératoires sont indiqués dans le tableau ci-après, ainsi que les différents rendements. Rappelons que TT signifie taux de transformation. C est le rapport entre la quantité de substrat considérée qui a disparue au cours de la réaction sur la quantité initiale. Ici, le rendement est calculé par rapport au benzylmercaptan mis initialement. Le RR est le rendement de réaction, c'est-à-dire la quantité de produit désirée, ramenée au substrat considéré initial, et RT est le rendement de transformation, c'est-à-dire la quantité de produit désirée obtenue, divisée par la quantité de substrat considéré ayant disparu.

Le tableau suivant résume les différents essais réalisés :

| Benzylmercaptan | Soude éq, mol, | Solvant | CTP 5% mol | Conc, Benzylmercaptan % p/p | R-22 éq, mol, | Temp, (°C) | TT (%) BT | RR(%) DFBS | RT (%) DFBS |
|---|---|---|---|---|---|---|---|---|---|
| 100 mmol | 2,5 broyée | TCB (1,2,4-trichlorobenzène | TDA-1 | 11 | 2 | 90 | 100 | 82 | 82 |
| « « | 2,5 broyée | « « | « « | 26 | 2 | 85 | 99,6 | 70 | 70 |
| « « | 2,5 sol 30% | « « | « « | 26 | 2 | 85 | 77 | 71 | 93 |
| « « | 1,1 sol 30% | « « | « « | 26 | 2 | 85 | 74 | 66 | 89 |
| « « | 1,1 sol 30% | « « | « « | 26 | 2 | 50 | 52 | 48 | 92 |
| « « | 1,1 sol 30% | « « | Pas de CTP | 26 | 2 | 90 | 67 | 59 | 87 |
| « « | 1,1 sol 30% | H₂O | Pas de CTP | 33 | 3-4 | 95 | 80,7 | 74,3 | 92 |
| « « | 1,1 sol 30% | H₂O | TDA-1 | 33 | 4 | 95 | 80,7 | 74,6 | 92 |
| « « | 1,1 KOH 50% | H₂O H₂O | Pas de CTP | 50 | 3 | 95 | 83 | 75 | 90 |
| « « | 2 NaOH sol 30% | H₂O | « « | 31 | 3 | 95 | 82 | 75 | 92 |
| 1,5 mol | 1,1 NaOH 30% | H₂O | « « | 33,4 | 1 P = 8,5 bar | 95 | 79 | 75,3 | 95 |

| | | | | | | | | | |
|---|---|---|---|---|---|---|---|---|---|
| Dans le tableau, BT signifie benzylthiol, c'est-à-dire le benzylmercaptan, Le DFBS signifie le sulfure de benzyle et de difluorométhyle. | | | | | | | | | |

### Exemple 3 - Exemple comparatif - Rôle de l'eau

Les conditions de l'essai 2k ont été reproduites en augmentant la quantité d'eau de manière significative de manière qu'il y ait 5 moles de benzylmercaptan par kg d'eau présente dans la phase. Le résultat obtenu montre une dégradation dramatique du rendement, à savoir les rendements de l'ordre de 15%.

### Exemple 4 - Essai de chloration

Dans un réacteur, 75 g d'eau sont chargés, puis 26,1 g de difluorométhylthiobenzyl sont additionnés.

Le milieu réactionnel est incolore biphasique. Le mélange est refroidi à 10°C et on introduit lentement le chlore (32 g) dans la masse.

Pour maintenir les 10°C, malgré la très forte exothermie, la consigne de température de double enveloppe est régulée à -5°C.

L'introduction de chlore est arrêtée lorsque l'exothermie cesse et que le milieu réactionnel commence à jaunir.

Le chlore est introduit en 3 heures (31,6 g exactement).

Au terme des 3 heures, on laisse le milieu réactionnel revenir à température ambiante, on effectue un balayage d'azote et on coupe l'agitation. Les deux phases décantent facilement. La phase aqueuse est limpide incolore. La phase organique est limpide jaune.

La phase organique est séchée sur MgSO₄ et filtrée sur fritté.

On récupère m = 37,6 g de phase liquide dont l'analyse par chromatographie en phase gazeuse indique un rendement chimique de 82% en chlorure de difluorométhanesulfonyle.

Après distillation, on obtient 14,3 g de chlorure de difluorométhanesulfonyle de pureté supérieure à 99% (point d'ébullition : 66°C sous 300 mbars).

## Revendications

1. Procédé de synthèse de dérivés à radical hydrogénofluorométhylène-sulfonyles comportant :
a) une étape de condensation dans un solvant d'un sulfure d'alcoyle dont l'alcolyle est soit tertiaire soit un aralcoyle de nature benzylique et d'un cation (thiolate) avec un composé présentant un carbone d'hybridation sp³ porteur d'un hydrogène, d'un fluor, d'un halogène lourd choisi parmi le chlore, le brome et ll'iode, et d'un groupe électro-attracteur choisi parmi le fluor et ceux dont le σₚ est au moins égal à 0,2, avantageusement à 0,4 ;
b) une étape d'oxydation, avantageusement d'halogénation, de préférence de chloration ou de bromation, en présence d'une phase aqueuse ;
ledit solvant de l'étape a) étant choisi parmi les solvants non miscibles à l'eau, parmi les phases aqueuses, et parmi la combinaison biphasique d'un solvant non miscible à l'eau et d'une phase aqueuse, lesdites phases aqueuses comportant au plus 1/3 en masse de solvant non aqueux miscible à l'eau ; le rapport entre la quantité en équivalent du sulfure d'alcoyle et la quantité d'eau en mole état au plus égale à 50.

2. Procédé selon la revendication 1, **caractérisé par le fait que** le milieu aqueux de l'étape a) comporte en outre une base forte de pKa de l'acide associé au moins égal à 10, avantageuse à 12, de préférence à 14, dont la quantité exprimée équivalent est avantageusement au moins égale à 5% la quantité dudit thiolate (R-S-M).

3. Procédé selon les revendications 1 et 2, **caractérisé par le fait que** le milieu aqueux de l'étape a) comporte en outre une base forte pKa, au plus égale à une fois la quantité dudit thiolate (R-S-M).

4. Procédé selon les revendications 1 à 3, **caractérisé par le fait qu'**à l'étape a), le rapport molaire entre la quantité en un éventuel solvant polaire exprimé en mole et la somme exprimée en équivalents des cocations du sulfure et de la base éventuelle est au plus égal 1, et avantageusement au plus égal 1/2, de préférence à 1/10.

5. Procédé selon les revendications 1 à 4, **caractérisé par le fait que** le groupe éiectro-attracteur est choisi parmi le fluor et les groupes Rf ; par Rf on entend un radical de formule :
GEA'-(CX₂)ₚ-
- où les X, semblables ou différents, représentent un chlore, un fluor ou un radical de formule CₙF₂ₙ₊₁, avec n entier au plus égal à 5, de préférence à 2, avec la condition qu'au moins un des X soit fluor, fluor avantageusement porté par le carbone relié au soufre ;
- où p représente un entier au plus égal à 2 ;
- où GEA' représente un groupe électro-attracteur (c'est à dire σₚ supérieur à zéro, avantageusement à 0,1, de préférence à 0,2) dont les éventuelles fonctions sont inertes dans les conditions de la réaction, avantageusement fluor ou un reste perfluoré de formule CₙF₂ₙ₊₁ avec n entier au plus égal à 8, avantageusement à 5.

6. Procédé selon les revendications 1 à 5, **caractérisé par le fait que** le nombre total de carbone de Rf est avantageusement compris entre 1 et 15, de préférence entre 1 et 10.

7. Procédé selon les revendications 1 à 6, **caractérisé par le fait que** le groupe électro-attracteur est le fluor et ledit composé est le chlorodifluorométhane (R-22).

8. Procédé selon les revendications 1 à 7, **caractérisé par le fait que** l'on fait réagir le sel de sodium de benzylmercaptan et le chlorodifluorométhane (R-22).

9. Procédé selon les revendications 1 à 8, **caractérisé par le fait que** le rapport entre l'eau, exprimée en mole, et le cation, exprimé en équivalent, est au moins égal à 4, avantageusement à 6, de préférence à 8.

10. Procédé selon les revendications 1 à 9, **caractérisé par le fait que** ledit cation est monovalent.

11. Procédé selon les revendications 1 à 10, **caractérisé par le fait que** ledit cation est choisi parmi le phosphonium, les ammoniums quaternaires et les alcalins, avantageusement ces derniers.

12. Procédé selon les revendications 1 à 11, **caractérisé par le fait que** lorsque l'on désire obtenir un halogénure d'acide, l'étape b) est menée, soit en présence d'un sel dissocié dissous dans le mélange réactionnel, soit en maintenant un pH dans l'intervalle allant de 4 à 9, avantageusement de 5 à 8, et lorsque l'on désire l'obtention d'un acide en milieu acide fort et peu salin.

13. Procédé selon les revendications 1 à 12, **caractérisé par le fait que** la température de réaction à l'étape a) est comprise entre 50°C et 110°C, de préférence entre 60°C et 90°C.

14. Procédé selon les revendications 1 à 13, **caractérisé par le fait que** l'halogénure de formule :
R Ξ
dans laquelle :
- R est soit un alcolyle tertiaire soit un aralcoyle de nature benzylique,
- et Ξ représente un halogène, de préférence le chlore et le brome, est utilisé pour former à nouveau un sulfure d'alcoyle.

## Claims

1. Process for the synthesis of derivatives comprising a hydrofluoromethylenesulphonyl radical comprising:
a) a stage of condensation in a solvent of a cationic alkyl sulphide (thiolate), the alkyl of which is either tertiary or an aralkyl of benzylic nature, with a compound exhibiting a carbon of sp³ hybridization carrying a hydrogen, a fluorine, a heavy halogen, chosen from chlorine, bromine and iodine, and an electron-withdrawing group chosen from fluorine and those for which the σₚ is at least equal to 0.2, advantageously to 0.4;
b) a stage of oxidation, advantageously of halogenation, preferably of chlorination or of bromination, in the presence of an aqueous phase;
said solvent of stage a) being chosen from water-immiscible solvents, from aqueous phases and from the two-phase combination of a water-immiscible solvent and of an aqueous phase, said aqueous phases comprising at most 1/3 by weight of water-miscible nonaqueous solvent; the ratio of the amount in equivalents of the alkyl sulphide to the amount in moles of water being at most equal to 50.

2. Process according to Claim 1, **characterized in that** the aqueous medium of stage a) additionally comprises a strong base with a pKa of the associated acid at least equal to 10, advantageously to 12, preferably to 14, the amount of which, expressed in equivalents, is advantageously at least equal to 5% of the amount of said thiolate (R-S-M).

3. Process according to Claims 1 and 2, **characterized in that** the aqueous medium of stage a) additionally comprises a strong base pKa at most equal to one times the amount of said thiolate (R-S-M).

4. Process according to 1 to 3, **characterized in that**, in stage a), the molar ratio of the amount of a possible polar solvent, expressed in moles, to the sum, expressed in equivalents, of the cocations of the sulphide and of the possible base is at most equal to 1 and advantageously at most equal to 1/2, preferably to 1/10.

5. Process according to Claims 1 to 4, **characterized in that** the electron-withdrawing group is chosen from fluorine and the Rf groups; the term "Rf" is understood to mean a radical of formula:
EWG'-(CX₂)ₚ-
- where the X groups, which are alike or different, represent a chlorine, a fluorine or a radical of formula CₙF₂ₙ₊₁, with n being an integer at most equal to 5, preferably to 2, with the condition that at least one of the X groups is fluorine, fluorine advantageously carried by the carbon connected to the sulphur;
- where p represents an integer at most equal to 2;
- where EWG' represents an electron-withdrawing group (that is to say, σₚ greater than zero, advantageously than 0.1, preferably than 0.2), the possible functional groups of which are inert under the conditions of the reaction, advantageously fluorine or a perfluorinated residue of formula CₙF₂ₙ₊₁ with n being an integer at most equal to 8, advantageously to 5.

6. Process according to Claims 1 to 5, **characterized in that** the total carbon number of Rf is advantageously between 1 and 15, preferably between 1 and 10.

7. Process according to Claims 1 to 6, **characterized in that** the electron-withdrawing group is fluorine and said compound is chlorodifluoromethane (R-22).

8. Process according to Claims 1 to 7, **characterized in that** the sodium salt of benzyl mercaptan and chlorodifluoromethane (R-22) are reacted.

9. Process according to Claims 1 to 8, **characterized in that** the ratio of the water, expressed in moles, to the cation, expressed in equivalents, is at least equal to 4, advantageously to 6, preferably to 8.

10. Process according to Claims 1 to 9, **characterized in that** said cation is monovalent.

11. Process according to Claims 1 to 10, **characterized in that** said cation is chosen from phosphonium, quaternary ammoniums and alkali metals, advantageously the latter.

12. Process according to Claims 1 to 11, **characterized in that**, when it is desired to obtain an acid halide, stage b) is carried out either in the presence of a dissociated salt dissolved in the reaction mixture or by maintaining a pH within the range from 4 to 9, advantageously from 5 to 8, and, when it is desired to obtain an acid, in a strong acid and not very saline medium.

13. Process according to Claims 1 to 12, **characterized in that** the reaction temperature in stage a) is between 50°C and 110°C, preferably between 60°C and 90°C.

14. Process according to Claims 1 to 13, **characterized in that** the halide of formula:
R-Ξ
in which:
- R is either a tertiary alkyl or an aralkyl of benzylic nature,
- and Ξ represents a halogen, preferably chlorine and bromine,
is used to reform an alkyl sulphide.

## Patentansprüche

1. Verfahren zur Synthese von Derivaten, die ein Fluorwasserstoff-Methylensulfonylradikal aufweisen, umfassend:
a) einen Schritt der Kondensation eines Sulfids von einem Alkyl, wobei des Alkyl entweder terhär oder ein Aralkyl vom Benzyltyp ist, und einem Kation (Thiolat) in einem Lösemittel mit einer Verbindung, die einen Kohlenstoff der sp³-Hybridisierung, der Wasserstoff, Fluor und schweres Halogen trägt, ausgewählt aus Chlor, Brom und Iod, und einer elektronenziehenden Gruppe aufweist, ausgewählt aus Fluor und jenen, deren σₚ mindestens gleich 0,2, vorteilhafterweise gleich 0,4 ist;
b) einen Schritt der Oxidation, vorteilhafterweise der Halogenierung, bevorzugt der Chlorierung oder der Bromierung in Gegenwart einer wässrigen Phase;
wobei das Lösemittel aus Schritt a) ausgewählt ist aus den nicht mit Wasser mischbaren Lösemitteln, aus den wässrigen Phasen und aus der zweiphasigen Kombination aus einem nicht mit Wasser mischbaren Lösemittel und einer wässrigen Phase, wobei die wässrigen Phasen höchstens 1/3 Masseanteile des mit Wasser mischbaren nicht wässrigen Lösemittels umfassen; wobei das Verhältnis zwischen der Alkylsulfidmenge in Äquivalenten und der Wassermenge in Mol höchstens gleich 50 ist.

2. Verfahren nach Anspruch 1, **gekennzeichnet durch** die Tatsache, dass das wässrige Medium aus Schritt a) ferner eine starke Base umfasst mit einer pKa der assoziierten Säure von mindestens gleich 10, vorteilhafterweise gleich 12, bevorzugt gleich 14, deren Menge, ausgedrückt in Äquivalenten, vorteilhafterweise mindestens gleich 5 % der Menge des Thiolats (R-S-M) ist.

3. Verfahren nach den Ansprüchen 1 und 2, **gekennzeichnet durch** die Tatsache, dass das wässrige Medium aus Schritt a) ferner eine starke Base umfasst, mit einer pKa von höchstens gleich ein Mal der Menge des Thiolats (R-S-M).

4. Verfahren nach den Ansprüchen 1 bis 3, **gekennzeichnet durch** die Tatsache, dass in Schritt a) das Molverhältnis zwischen der Menge an einem optionalen polaren Lösemittel, ausgedrückt in Mol, und der Summe, ausgedrückt in Äquivalenten, der Verkokungen des Sulfids und der optionalen Base höchstens gleich 1, und vorteilhafterweise höchstens gleich 1/2, bevorzugt gleich 1/10 ist.

5. Verfahren nach den Ansprüchen 1 bis 4, **gekennzeichnet durch** die Tatsache, dass die elektronenziehende Gruppe aus Fluor und den Rf-Gruppen ausgewählt ist; unter Rf ist ein Radikal der folgenden Formel zu verstehen:
GEA'-(CX₂)ₚ-
- wobei die X, gleich oder verschieden, für Chlor, Fluor oder ein Radikal der Formel CₙF₂ₙ₊₁ stehen, worin die ganze Zahl n höchstens gleich 5, bevorzugt gleich 2 ist, unter der Bedingung, dass mindestens eines der X Fluor ist, wobei das Fluor vorteilhafterweise von dem Kohlenstoff getragen wird, der mit dem Schwefel verknüpft ist;
- wobei p für eine ganze Zahl steht, die höchstens gleich 2 ist;
- wobei GEA' für eine elektronenziehende Gruppe (das heißt, σₚ größer als null, vorteilhafterweise größer als 0,1, bevorzugt größer als 0,2) steht, deren optionale Funktionen unter den Reaktionsbedingungen inert sind, vorteilhafterweise für Fluor oder einen perfluorierten Rest der Formel CₙF₂ₙ₊₁ steht, worin die ganze Zahl n höchstensgleich 8, vorteilhafterweise gleich 5 ist.

6. Verfahren nach den Ansprüchen 1 bis 5, **gekennzeichnet durch** die Tatsache, dass die Gesamtzahl an Kohlenstoff von Rf vorteilhafterweise im Bereich zwischen 1 und 15, bevorzugt zwischen 1 und 10 liegt.

7. Verfahren nach den Ansprüchen 1 bis 6, **gekennzeichnet durch** die Tatsache, dass die elektronenziehende Gruppe Fluor ist und die Verbindung Chlordifluormethan (R-22) ist.

8. Verfahren nach den Ansprüchen 1 bis 7, **gekennzeichnet durch** die Tatsache, dass man das Natriumsalz von Benzylmercaptan und Chlordifluormethan (R-22) reagieren lässt.

9. Verfahren nach den Ansprüchen 1 bis 8, **gekennzeichnet durch** die Tatsache, dass das Verhältnis zwischen Wasser, ausgedrückt in Mol, und dem Kation, ausgedrückt in Äquivalenten, mindestens gleich 4, vorteilhafterweise 6, bevorzugt 8 beträgt.

10. Verfahren nach den Ansprüchen 1 bis 9, **gekennzeichnet durch** die Tatsache, dass das Kation monovalent ist.

11. Verfahren nach den Ansprüchen 1 bis 10, **gekennzeichnet durch** die Tatsache, dass das Kation aus Phosphonium, den quartären Ammoniumverbindungen und alkalischen Verbindungen, vorteilhafterweise letztgenannten, ausgewählt ist.

12. Verfahren nach den Ansprüchen 1 bis 11, **gekennzeichnet durch** die Tatsache, dass, wenn es gewünscht wird, ein Säurehalogenid zu erhalten, Schritt b) entweder in Gegenwart eines dissoziierten Salzes ausgeführt wird, das in der Reaktionsmischung gelöst ist, oder indem ein pH-Wert in einem Intervall gehalten wird, das von 4 bis 9, vorteilhafterweise von 5 bis 8 reicht, und wenn der Erhalt einer Säure in stark saurem und wenig salzigem Medium gewünscht wird.

13. Verfahren nach den Ansprüchen 1 bis 12, **gekennzeichnet durch** die Tatsache, dass die Reaktionstemperatur in Schritt a) im Bereich zwischen 50°C und 110 °C, bevorzugt zwischen 60 °C und 90 °C liegt.

14. Verfahren nach den Ansprüchen 1 bis 13, **gekennzeichnet durch** die Tatsache, dass das Halogenid der Formel:
RΞ
worin:
- R entweder ein tertiäres Alkyl oder ein Aralkyl vom Benzyltyp ist,
- und Ξ für ein Halogen, bevorzugt Chlor und Brom steht,
verwendet wird, um erneut ein Sulfid von Alkyl zu bilden.
